(19) Europäisches Patentamt
European Patent Office
Office européen des brevets

(11) Veröffentlichungsnummer : **0 097 244**
**B1**

(12) # EUROPÄISCHE PATENTSCHRIFT

(45) Veröffentlichungstag der Patentschrift : **02.01.86**

(51) Int. Cl.$^4$ : **C 07 C120/00**, C 07 C121/34

(21) Anmeldenummer : **83104836.8**

(22) Anmeldetag : **17.05.83**

(54) **Verfahren zur Herstellung von Cyanacetaldehyd-acetalen.**

(30) Priorität : **16.06.82 DE 3222519**

(43) Veröffentlichungstag der Anmeldung : **04.01.84 Patentblatt 84/01**

(45) Bekanntmachung des Hinweises auf die Patenterteilung : **02.01.86 Patentblatt 86/01**

(84) Benannte Vertragsstaaten : **BE CH DE FR GB IT LI NL**

(56) Entgegenhaltungen :
**HOUBEN-WEYL: "Methoden der organischen Chemie", Band VI/3, Sauerstoffverbindungen 1, Teil 3, 1965, Seiten 229-232, 268, Georg Thieme Verlag, Stuttgart, DE**
**JOURNAL OF THE AMERICAN CHEMICAL SOCIETY, Band 69, November 1947, Seiten 2657-2660, S.M. McELVAIN et al.: "The preparation, alcoholysis and reduction of cyanoacetaldehyde diethylacetal. Malonaldehyde tetraethylacetal"**

(73) Patentinhaber : **DYNAMIT NOBEL AKTIENGESELLSCHAFT**
**Postfach 1209**
**D-5210 Troisdorf, Bez. Köln (DE)**

(72) Erfinder : **Theis, Christoph, Dr.**
**Hoher Rain 5**
**D-5216 Niederkassel-Rheidt (DE)**
Erfinder : **Fickert, Gudrun**
**Ravensbergerweg 5**
**D-5210 Troisdorf (DE)**

**EP 0 097 244 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung beschreibt ein kontinuierlich ausführbares Verfahren zur Herstellung von ggf. substituierten Cyanacetaldehyd-acetalen durch Umsetzung von ggf. α-C-substituierten β-Alkoxyacrylnitrilen mit Alkoholen in Gegenwart katalytischer Mengen einer geeigneten Base bei erhöhter Temperatur.

Nach Am. Chem. Soc. 69 (1947) Seiten 2657/2660 führt die Behandlung von β-Ethoxyacrylnitril mit Alkohol in Gegenwart kleiner Mengen von Natrium Ethoxid durch quantitative Addition des Alkohols zur Bildung des Cyanoacetals. Nach Erhitzen dieser Lösung des Cyanoacetals mit einigen Tropfen konzentrierter Schwefelsäure wird Alkohol und der Ausgangsstoff β-Ethoxyacrylnitril in Mengen von 40 % gefunden.

Nach eigenen Untersuchungen ist die Reaktion unvollständig und die Umsetzung erfordert bereits im Maßstab von 100 g hohen Zeitaufwand, der bei Übergang zum Maßstab von 1 kg nicht sinkt, wobei sich bei größeren Mengen entscheidend die Reinheit der Produkte vermindert.

Weitere Herstellwege, beispielsweise durch Umsetzung von Bromacetaldehydacetal mit Alkalicyaniden oder von β-Chloracrylnitril mit Natriumalkoholat/Alkoholen sind schwierig ausführbar, haben schwer zugängliche Ausgangsstoffe und erzielten mäßige Ausbeuten.

Diese bekannten Herstellwege sind zudem nur diskontinuierlich ausführbar, erforden hohen Aufwand bei der Aufarbeitung der Reaktionslösungen z. B. für Neutralisationen und Abtrennen der Feststoffe und stellen hohe Anforderungen an Sicherheitseinrichtungen und ökologische Schutzmaßnahmen.

Es bestand daher die Aufgabe, das Zielprodukt der Formel I nach einem wirtschaftlichen und technisch einfach ausführbaren Verfahren herzustellen. Weiter war das Ziel, die Umsetzung praktisch vollständig in angemessen kurzer Zeit auszuführen und das Produkt als reinen Stoff zu gewinnen.

Gegenstand der Erfindung ist daher das Verfahren gemäß Patentanspruch 1.

Die Substituenten $R^1$ und $R^2$ sind allgemein solche, die sich bei der Umsetzung inert verhalten. Die Ausgangsstoffe II tragen bevorzugt Alkylreste, von dener die mit 1 bis 3 C-Atomen, insbesondere der Ethyl- und der Methylrest sehr bevorzugt sind. Der Rest $R^2$ hat sehr bevorzugt die Bedeutung H, weiter bevorzugt Methyl, Ethyl, Phenyl oder Benzyl. Soweit der Rest Cycenthalten ist, sind isocyclische oder heterocyclische Ringsysteme mit 5 oder 6 Ringgliedern bevorzugt, wobei isocyclische d. h. nur Kohlerstoff als Ringglied enthaltende Ringe besonders die von Benzol. Cyclopentan bis Cycloheptan sind, heterocyclische d. h. neben Kohlenstoff weitere ringbildende Atome enthaltende Ringe als Heteroatome u. a. O, N, S, P enthalten, besonders einkernige Ringe mit 1 oder 2 O, N oder S neben O sind.

Der Rest $R^1$ ist bevorzugt ein Alkylrest mit 1 bis 3 C-Atomen, insbesondere Ethyl oder Methyl oder der Rest eines Diels, besonders der der Ethylenglykols bis Butylenglykols wobei für ggf. unterbrechende Heteroatome das gesagte gilt. Der Rest $R^1$ im basischer Katalysator soll, bis auf Ausnahmen, mit dem Rest $R^1$ des Alkohols übereinstimmen. Der Rest $R^3$ ist bevorzugt Ethyl oder Methyl.

Soweit einer der Reste $R^1$ oder beide Reste $R^1$ die eines gesättigten oder ungesättigten Diols sind, wird bevorzugt die Reaktion soweit geführt, daß dieser Rest $R^1$ erhalten bleibt und, wenn gewollt, in einer zweiten Stufe das einen Alkylen- bzw. Alkenylenrest enthaltende cyclische Acetal gebildet.

Verfahrensgemäß wird bevorzugt der basische Katalysator im Alkohol gelöst dem Reaktionsgefäß zugeführt.

Weiter werden bevorzugt die Nitrilkomponente II und die den Katalysator IV enthaltende Alkoholkomponente III auf getrennten Wegen gleichzeitig dem Reaktor zugeführt.

Drittens werden bevorzugt die Reaktionskomponenten gut gemischt und möglichst schnell durch Erwärmen zur Reaktion gebracht.

Als Metall M im Alkoholat ist Na sehr bevorzugt, K und Ca weiterhin bevorzugt.

Sehr bevorzugt werden die Reaktionskomponenten unmittelbar an der Eintrittsstelle gründlich durchmischt, wonach diese Reaktionsmischung eine durch einen Thermostaten auf die Reaktionstemperatur gehaltene Reaktionszone in einer festgelegten mittleren Verweilzeit durchfließt und abgekühlt wird.

Die mittlere Verweilzeit ist dabei der Quotient aus Füllvolumen des Reaktors und der Summe der Volumnia der Ausgangsstoffe bei 20 °C, die pro Stunde in den Reaktor eingeschleust werden.

Durch ein solches kontinuierliches Verfahren, vorzugsweise in einem Durchflußreaktor werden die angestrebten Umsätze und Ausbeuten erzielt und Nebenreaktionen sehr weitgehend unterdrückt.

Die Reaktionskomponenten können bei Umgebungstemperatur in den Reaktor eingeschleust werden, jedoch wird es zur Erzielung minimaler Verweilzeiten bevorzugt, die Komponenten auf annähernd Reaktionstemperatur vorzuheizen und dann in den Reaktor einzuschleusen.

Entscheidend für die Durchführung des kontinuierlichen Verfahrens und dessen Vorteile ist die kontinuierliche Einschleusung des basischen Katalysators in die Reaktionszone, was am einfachsten durch die bevorzugte Zuführung der Lösung des Katalysators im verwendeten Alkohol erfolgt.

Die Desaktivierung des Katalysators nach der Umsetzung kann, wie vielfach zur Zersetzung von Alkoholaten üblich, durch Neutralisation mit Eisessig erfolgen. Das ergibt jedoch Nachteile im vorliegenden nichtwässrigen Milieu durch Verfälschung der pH-Wert-Messung und besonders durch Bildung einer sehr schwer handhabbaren hochviskosen Suspension aus der Reaktionslösung.

Daher erfolgt erfindungsgemäß sehr bevorzugt die Desaktivierung des Katalysators mit Kohlendioxid

oder ggf. mit einer anorganischen oder organischen Säure. Neben dem bevorzugten Kohlendioxid können starke oder mittelstarke Säuren wie Schwefelsäure, HCl-Gas, Phosphorsäure oder z. B. Ameisensäure verwendet werden.

Die Aufarbeitung erfolgt, ggf. nach Abtrennung des Feststoffs, ganz oder teilweise durch Destillation unter vermindertem Druck.

Die Desaktivierung kann nach Abkühlung der Reaktionsmischung auf Raumtemperatur im Falle des Kohlendioxids chargenweise oder kontinuierlich durch Begasung mit $CO_2$ oder Zugabe von $CO_2$ unter Druck bei guter Rührung vorgenommen werden. Die Abtrennung der bei der Katalysatorinaktivierung entstehenden Na-Salze der den eingesetzten Alkoholen entsprechenden Kohlensäurehalbester kann unter $CO_2$-Atmosphäre durch Filtrieren oder Zentrifugieren erfolgen, erfolgt jedoch bevorzugt durch fraktionierte Destillation bei vermindertem Druck, beispielsweise im Bereich 0,1 bis 500 mbar, ggf. unter Verwendung einer Fraktionierkolonne zusammen mit der destillativen Aufarbeitung der Reaktionsmischung. Hierbei ist die Begasung der Reaktionssuspension bzw. der vom Feststoff befreiten Reaktionslösungen mit $CO_2$ während der destillativen Aufarbeitung entscheidend für Reinheit und Ausbeute. Der nicht zur Reaktion verbrauchte und abdestillierte Alkohol wird mit neuem Katalysator ergänzt und in den Kreislauf zurückgeführt. Hochreine Produkte werden erhalten, wenn man entsprechend der thermischen Instabilität der hergestellten Cyanacetaldehydacetale eine Destillation bei vermindertem Druck im Bereich von 0,1 bis 50 mbar ausführt. Eine so erhaltene Vorfraktion der eigentlichen Produktfraktion enhalten das bei der Alkoholaddition ggf. nicht umgesetzte β-Alkoxyacrylnitril sowie das infolge thermischer Spaltung sich rückbildende β-Alkoxy-nitril neben großen Anteilen von Produkt.

Diese Vorfraktionen können, ggf. mit β-Alkoxyacrylnitril vermischt in den Kreislauf zurückgeführt werden.

Das Mol-Verhältnis der Nitrilkomponente II zum Alkohol soll im Bereich 1 : 1 bis 1 : 10, vorzugsweise 1 : 1,1 bis 1 : 2,5, liegen.

Die Temperatur im Reaktor liegt zwischen 30 °C und bis max. ca. 5 °C unter der Siedetemperatur des verwendeten Alkohols vorzugsweise jedoch im Bereich 50 bis 100 °C, bei niedrigsiedenden Alkoholen im Bereich 50 bis 62 °C. Die Temperatur der einzuschleusenden Edukte ist nicht kritisch, soll jedoch zur Verminderung der mittleren Verweilzeit vorzugsweise nahe der späteren Reaktionstemperatur liegen.

Die Katalysator-Konzentration soll im Bereich 0,05 bis 15 Gew.-%, vorzugsweise 0,5 bis 2,5 Gew.-%, bezogen auf das Gewicht des eingesetzten Alkohols, liegen.

Die Nitrilkomponente kann in unverdünnter Form oder verdünnt mit dem verwendeten Alkohol eingesetzt werden.

Es wird überraschend erreicht, daß die mittlere Verweilzeit im Durchflußreaktor im Bereich 2 bis 120 Minuten, vorzugsweise 5 bis 60 Minuten, liegt.

Wichtig ist, daß die destillative Aufarbeitung, die Abtrennung des Alkohols und die Produkt-Reindestillation immer dann im Vakuum erfolgt, wenn andernfalls eine Zersetzung des Produkts zu befünchten ist, gleichgültig ob die Destillation kontinuierlicher Verfahrensweise oder im Batch-Verfahren erfolgt.

Der mit hoher Reinheit anfallende Alkohol wird in den Prozeß zurückgeführt.

Die hergestellten Cyanacetaldehydacetale sind wertvolle Ausgangsstoffe für die Synthese zahlreicher pharmazeutischer Verbindungen, wie beispielsweise in 5-Aryl-substituierte 2,4-Diamino-pyrimidine (Trimethoprin) (DE-OS 30 14 412).

## Beispiel 1

In einen Durchflußreaktor, bestehend aus einem beheizbaren 250 ml-Vierhalskolben mit Doppel-Einleitungsrohr, Rührer, Innenthermometer und aufgesetztem, thermostatisiertem Intensivkühler als Wärmetauscher, werden mittels getrennter Pumpen die bei Raumtemperatur 3-Ethoxy-Propennitril und 1,5 Gew.-%ige ethanolische Natriumethoxid-Lösung unter guter Rührung kontinuierlich eindosiert. Nach Einsetzen der Reaktion wird die Reaktor-Innentemperatur durch Heizen bzw. Kühlen auf 60 °C gehalten. Es werden je Stunde 417,4 g entsprechend 441,7 ml 3-Ethoxy-propennitril und 341,0 g entsprechend 432 ml Alkoholatlösung eingeschleust. Die mittlere Verweilzeit gemäß der vorstehenden Definition beträgt 55 Minuten.

Die am Kopf des Reaktors austretende Reaktionsmischung wird mittels eines absteigenden Produkt-Kühlers kontinuierlich auf Raumtemperatur abgekühlt, in diese abgekühlte Lösung wird bei intensiver Rührung ein gleichmäßiger, schwachen Strom trockenen Kohlendioxides eingeleitet.

Aus dem gaschromatographischen Analyse der Reaktionssuspension ergibt sich ein Umsatz mehr als 99,2 %, bezogen auf eingesetztes 3-Ethoxy-Propennitril.

343,4 g dieser Reaktions-Suspension werden unter schwachem $CO_2$-Strom bei Normaldruck und anschließend bei 15 mbar an einer 30 cm-VIGREUX-Kolonne destilliert. Man erhält 63,5 g Ethanol, das in den Prozeß zurückgeführt wird, 20,3 g als Vorlauf der Vakuumdestillation und 251,2 g 3,3-Diethoxipropannitril. Als Destillations-Rückstand verbleiben 5,8 g. Der Vorlauf enthält nach der gaschromotographen Analyse 95,0 Flä-% Zielprodukt und wird in den Durchflußreaktor recyclisiert.

Die Produkt-Hauptfraktion weist eine Reinheit von mehr als 99 % auf. Die Gesamt-Ausbeute an Produkt beträgt 96,1 % d. Th.

Beispiel 2

Analog Beispiel 1 werden nunmehr vorgeheizte Reaktion-Komponente in den Reaktor eingeschleust, nämlich

669,4 g 1,5 Gew.-%ige ethanolische Natriumethoxid-Lösung von 46 °C und
539,8 g 3-Ethoxy-propennitril vorgeheizt auf 50 °C je Stunde.
Mittlere Verweilzeit : 31,5 Minuten.
Die Katalysator-Desaktivierung und Aufarbeitung der Reaktionssuspension erfolgt wie in Beispiel 1.
Umsatz (gaschromatographisch) : mehr als 99,5 %, bezogen auf II.
Ausbeute : 96,8 % d. Th. (I bezogen auf II)
Reinheit der Hauptfraktion : mehr als 99,0 %.

**Patentansprüche**

1. Verfahren zur Herstellung von Cyanacetaldehyd-acetalen der Formel

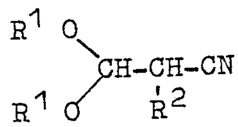

(I)

worin $R^1$ gleiche oder verschiedene geradkettige oder verzweigte Alkylreste mit 1 bis 20 C-Atomen, oder $—(CH_2)_m—$Cyc mit Cyc = isocyclische oder heterocyclische, einkernige oder mehrkernige aromatische oder cycloaliphatische Ringsysteme, welche gegebenenfalls Substituenten an den Ringen tragen, mit m = 0 bis 5, bedeuten oder einer der Reste $R^1$ oder beide Reste, $R^1$ die Bedeutung $—(CH_2)_p—OH$ oder $—(\text{alkenylen})_p—OH$ mit p = 2 bis 6, die gegebenenfalls durch ein oder mehrere Heteroatome unterbrochen sind, hat, oder beide Reste $R^1$ gemeinsam einen Alkylen- oder Alkenylen-Rest mit 2 bis 6 C-Atomen, der gegebenenfalls durch ein oder mehrere Heteroatome unterbrochen ist, bilden und worin $R^2$ die Bedeutung H, $R^1$, geradkettige oder verzweigte Reste $—(CH_2)_m—CN$, $—(CH_2)_m—OR^3$ oder $—(CH_2)_m—CH(OR^3)_2$ mit $R^3$ = Alkylreste mit 1 bis 12 C-Atomen und m bzw. Cyc in der vorstehenden Bedeutung, hat, dadurch gekennzeichnet, daß Verbindungen der Formel

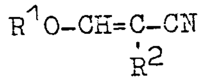

(II)

worin $R^1$ und $R^2$ die vorstehende Bedeutung haben, mit Alkoholen der Formel

$$R^1—OH \qquad (III)$$

worin $R^1$ die genannte Bedeutung hat im Mol-Verhältnis von II zu Alkohol im Bereich von 1 : 1 bis 1 : 10, in Gegenwart eines basischen Katalysators der Formel

$$M(OR^1)_n \qquad (IV)$$

worin M ein Alkalimetall mit n = 1 oder ein Erdalkalimetall mit n = 2 und $R^1$ die vorstehende Bedeutung hat, wobei der Katalysator im Alkohol in Konzentration von 0,05 bis 15 Gew.-% gelöst oder suspendiert ist, bei Temperaturen von 30 °C bis maximal 5 °C unter der Siedetemperatur des verwendeten Alkohols umgesetzt werden und der Katalysator nach erfolgter Reaktion durch Zugabe von Kohlendioxid unter Druck oder von anorganischen oder organischen Säuren desaktiviert wird, und die Aufarbeitung, ggf. nach Abtrennung des Feststoffs, ganz oder teilweise durch Destillation unter vermindertem Druck erfolgt.

2. Verfahren nach Anspruch 1, dadurch gekennzeichnet, daß das Verfahren in einem Durchflußreaktor mit einer bestimmten mittleren Verweilzeit kontinuierlich durchgeführt wird.

3. Verfahren nach Anspruch 2, dadurch gekennzeichnet, daß die mittlere Verweilzeit im Bereich 2 bis 120 Minuten, vorzugsweise 5 bis 60 Minuten, liegt.

4. Verfahren nach mindestens einem der Ansprüche 1 bis 3, dadurch gekennzeichnet, daß die Nitrilkomponente und die Alkoholkomponente getrennt und kontinuierlich in den Reaktor eingeschleust werden.

5. Verfahren nach Anspruch 4, dadurch gekennzeichnet, daß die Nitrilkomponente und die Alkoholkomponente vor Eintritt in den Reaktor auf die spätere ungefähre bis gleiche Reaktionstemperatur vorgeheizt werden.

6. Verfahren nach mindestens einem der Ansprüche 1 bis 5, dadurch gekennzeichnet, daß die Reaktionstemperatur im Bereich von 50 bis 100 °C liegt.

7. Verfahren nach mindestens einem der Ansprüche 1 bis 6, dadurch gekennzeichnet, daß das Molverhältnis der Nitrilkomponente und der Alkoholkomponente im Bereich 1 : 1,1 bis 1 : 2,5 liegt.

8. Verfahren nach mindestens einem der Ansprüche 1 bis 7, dadurch gekennzeichnet, daß der basische Katalysator kontinuierlich in Alkohol gelöst in den Reaktor eingeschleust wird.

9. Verfahren nach mindestens einem der Ansprüche 1 bis 8, dadurch gekennzeichnet, daß die Katalysatorkonzentration im Bereich 0,5 bis 2,5 Gew.-%, bezogen auf die Menge des eingesetzten Alkohols, liegt.

10. Verfahren nach einem der Ansprüche 1 bis 9, dadurch gekennzeichnet, daß die Katalysator-Desaktivierung kontinuierlich oder im Batch-Verfahren vorgenommen wird.

11. Verfahren nach Anspruch 1, dadurch gekennzeichnet, daß die destillative Aufarbeitung in Kohlendioxid-Atmosphäre erfolgt.

12. Verfahren nach mindestens einem der Ansprüche 1 bis 11, dadurch gekennzeichnet, daß der nicht umgesetzte und bei der destillativen Aufarbeitung anfallende Alkohol in das Reaktionsgefäß zurückgeführt wird.

13. Verfahren nach mindestens einem der Ansprüche 2 bis 12, dadurch gekennzeichnet, daß die ggf. bei der fraktionierten Destillation des Produktes anfallende Vorfraktion, die nicht umgesetztes Ausgangsmaterial und Produkt enthält, mit der Nitrilkomponente vermischt, in den Kreislauf zurückgeführt wird.

## Claims

1. Process for the production of cyanoacetaldehyde-acetals of the formula

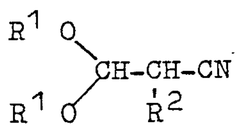

(I)

wherein $R^1$ denotes like or different straight-chained or branched alkyl residues with 1 to 20 C-atoms or $-(CH_2)_m-Cyc$ with Cyc = isocyclic or heterocyclic mononuclear or polynuclear aromatic or cycloaliphatic ring systems, which optionally carry substituents on the rings, with m = 0 to 5, or one of the residues $R^1$ or both residues $R^1$ has the meaning $-(CH_2)_p-OH$ or $-(\text{alkenylene})_p-OH$ with p = 2 to 6, which are optionally interrupted with one or several hetero atoms, or both residues $R^1$ form together an alkylene or alkenylene residue with 2 to 6 C-atoms, which is optionally interrupted by one or several hetero atoms, and wherein $R^2$ has the meaning H, $R^1$, straight-chained or branched residues $-(CH_2)_m-CN$, $-(CH_2)_m-OR^3$ or $-(CH_2)_m-CH(OR^3)_2$ with $R^3$ = alkyl residues with 1 to 12 C-atoms and m and Cyc respectively having the preceding meaning, characterised in that compounds of the formula

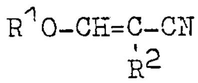

(II)

wherein $R^1$ and $R^2$ have the preceding meaning are reacted with alcohols of the formula

$$R^1-OH \quad \text{(III)}$$

wherein $R^1$ has the indicated meaning, in the molar ratio of II to alcohol in the range of 1 : 1 to 1 : 10 in the presence of a basic catalyst of the formula

$$M(OR^1)_n \quad \text{(IV)}$$

wherein M is an alkali metal with n = 1 or an alkaline earth metal with n = 2 and $R^1$ has the preceding meaning, with the catalyst being dissolved or suspended in the alcohol in a concentration of 0.05 to 15 % by weight, at temperatures of 30 °C to a maximum of 5 °C below the boiling temperature of the alcohol used, and the catalyst is deactivated, after achieving reaction, by addition of carbon dioxide under pressure or of inorganic or organic acids, and the working up, optionally after separation of the solids, takes place completely or partially by distillation under reduced pressure.

2. Process according to claim 1, characterised in that the process is carried out continuously in a percolation reactor with a specific average residence time.

3. Process according to claim 2, characterised in that the average residence time is in the region of 2 to 120 minutes, preferably 5 to 60 minutes.

4. Process according to at least one of claims 1 to 3, characterised in that the nitrile component and the alcohol are separately and continuously charged to the reactor.

5. Process according to claim 4, characterised in that the nitrile component and the alcohol

component are preheated before entry to the reactor to what corresponds approximately to a temperature equal to the later reaction temperature.

6. Process according to at least one of claims 1 to 5, characterised in that the reaction temperature is in the region of 50 to 100 °C.

7. Process according to at least one of claims 1 to 6, characterised in that the molar ratio of the nitrile component and the alcohol component lies in the region of 1 : 1.1 to 1 : 2.5.

8. Process according to at least one of claims 1 to 7, characterised in that the basic catalyst is continuously charged to the reactor dissolved in alcohol.

9. Process according to at least one of claims 1 to 8, characterised in that the catalyst concentration is in the region of 0.5 to 2.5 % by weight related to the amount of the alcohol employed.

10. Process according to at least one of claims 1 to 9, characterised in that the catalyst deactivation is carried out continuously or in a batch process.

11. Process according to claim 1, characterised in that the distillative working up takes place in a carbon dioxide atmosphere.

12. Process according to at least one of claims 1 to 11, characterised in that the alcohol which is unreacted and is obtained in the distillative working up is led back to the reaction vessel.

13. Process according to at least one of claims 2 to 12, characterised in that the first runnings which are possibly obtained in the fractional distillation of the product, which contain unreacted starting material and product, are led back, mixed with the nitrile component into the circulation.

**Revendications**

1. Procédé de préparation de cyanacétaldéhyde-acétals de formule

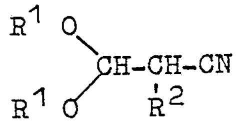

(I)

dans laquelle $R^1$ représente des restes alkyles à chaîne droite ou ramifiée, identiques ou différents, avec 1 à 20 atomes de C ou $-(CH_2)_m-$Cyc avec Cyc = systèmes cycliques ou hétérocycliques, aromatiques mononucléaires ou polynucléaires ou cyclaniques, éventuellement porteurs de substituants sur les noyaux, avec m = 0 à 5, ou un des restes $R^1$ ou les deux restes $R^1$ ont la signification $-(CH_2)_p-OH$ ou —(alcénylène)$_p$—OH avec p = 2 à 6, qui sont éventuellement interrompus par un ou plusieurs hétéro-atomes, ou les deux restes $R^1$ forment conjointement un reste alkylène ou alcénylène avec 2 à 6 atomes de C, éventuellement interrompus par un ou plusieurs hétéro-atomes et dans laquelle $R^2$ a la signification de H, de $R^1$, des restes $-(CH_2)_m-CN$, $-(CH_2)_m-OR^3$ ou $-(CH_2)_m-CH(OR^3)_2$ à chaîne droite ou ramifiée avec $R^3$ = restes alkyles avec 1 à 12 atomes de C et m, respectivement Cyc avec la signification précédente, caractérisé en ce que des composés de formule

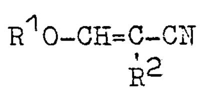

(II)

dans laquelle $R^1$ et $R^2$ ont la signification précédente, sont mis à réagir avec des alcools de formule

$$R^1-OH \quad (III)$$

où $R^1$ a la signification indiquée, dans un rapport molaire de II à alcool allant de 1 : 1 à 1 : 10 en présence d'un catalyseur basique de formule

$$M(OR^1)_n \quad (IV)$$

dans laquelle M représente un métal alcalin avec n = 1 ou un métal alcalino-terreux avec n = 2 et $R^1$ a la signification précédente, le catalyseur étant dissous ou en suspension dans l'alcool à une concentration de 0,05 à 15 % en poids, à des températures de 30 °C jusqu'à 5 °C au plus au-dessous de la température d'ébullition de l'alcool employé, que la réaction une fois effectuée, le catalyseur est désactivé par introduction d'anhydride carbonique sous pression ou d'acides inorganiques ou organiques et que le traitement, éventuellement après séparation des matières solides, s'effectue en totalité ou en partie par distillation sous pression réduite.

2. Procédé selon la revendication 1, caractérisé en ce que le procédé est mis en œuvre en continu dans un réacteur à circulation avec un temps de séjour moyen défini.

3. Procédé selon la revendication 2, caractérisé en ce que le temps de séjour moyen se situe dans le domaine de 2 à 120 minutes, préférablement de 5 à 60 minutes.

4. Procédé selon l'une quelconque des revendications 1 à 3, caractérisé en ce que le constituant nitrile et le constituant alcoolique sont chargés dans le réacteur, séparément et en continu.

5. Procédé selon la revendication 4, caractérisé en ce que, avant introduction dans le réacteur, le constituant nitrile et le constituant alcoolique sont préchauffés jusqu'à la température de réaction approximative à égale ultérieure.

6. Procédé selon l'une quelconque des revendications 1 à 5, caractérisé en ce que la température de réaction se situe dans le domaine de 50 à 100 °C.

7. Procédé selon l'une quelconque des revendications 1 à 6, caractérisé en ce que le rapport molaire du constituant nitrile au constituant alcoolique se situe dans le domaine de 1 : 1,1 à 1 : 2,5.

8. Procédé selon l'une quelconque des revendications 1 à 7, caractérisé en ce que le catalyseur basique est chargé en continu dans le réacteur, en solution dans de l'alcool.

9. Procédé selon l'une quelconque des revendications 1 à 8, caractérisé en ce que la concentration du catalyseur se situe dans le domaine de 0,5 à 2,5 % en poids, par rapport à la quantité de l'alcool mis en œuvre.

10. Procédé selon l'une quelconque des revendications 1 à 9, caractérisé en ce que la désactivation du catalyseur s'effectue selon un procédé en continu ou en discontinu.

11. Procédé selon la revendication 1, caractérisé en ce que le traitement distillatoire a lieu en atmosphère d'anhydride carbonique.

12. Procédé selon l'une quelconque des revendications 1 à 11, caractérisé en ce que l'alcool non transformé et provenant du traitement distillatoire est renvoyé dans le réacteur.

13. Procédé selon l'une quelconque des revendications 2 à 12, caractérisé en ce que la fraction de tête se formant éventuellement lors de la distillation fractionnée du produit, qui contient de la matière de départ n'ayant pas réagi et le produit, est recyclée, en mélange avec le constituant nitrile.